# EUROPEAN PATENT APPLICATION

(11) **EP 3 950 689 A1**
(43) Date of publication of application: **09.02.2022**
(21) Application number: 20777026.4
(22) Date of filing: 25.03.2020
(51) Int. Cl.: C07D 473/10, C07D 473/08, C07D 473/06, C07D 473/02, C07D 239/553, A61P 29/00, A61P 1/18, A61P 19/02, A61P 1/16, A61K 31/513, A61K 31/522

(54) **NUCLEOSIDE DERIVATIVE FOR PREVENTING AND TREATING INFLAMMATION AND APPLICATION THEREOF**

(30) Priority: 27.03.2019 CN 201910239233
(71) Applicant: West China Hospital of Sichuan University, Chengdu, Sichuan 610041 (CN)
(72) Inventor: HUANG, Wen, Chengdu, Sichuan 610047 (CN)
(74) Representative: Vesterinen, Jussi Tapio
(86) International application number: PCT/CN2020/080993
(87) International publication number: WO 2020/192665

(57) **Abstract**

The present invention provides a nucleoside derivative for preventing and treating inflammation and an application thereof in the preparation of a drug for preventing and treating inflammatory diseases. The nucleoside derivative for preventing and treating inflammation provided by the present invention can significantly ameliorate conditions of pancreatitis, hepatitis, arthritis and the like, ameliorate organ injury and inflammatory indexes, and have better effects than positive control drug, indometacin. Compared with the conventional anti-inflammatory drugs, aspirin, ibuprofen, indomethacin, phenylbutazone, diclofenac, piroxicam and glucocorticoids, the nucleoside derivative for preventing and treating inflammation provided by the present invention has the advantage of significantly fewer side effects.

## Description

### TECHNICAL FIELD

The present invention belongs to the field of medicines, and relates to a nucleoside derivative for preventing and treating inflammation and an application thereof in preparation of a drug for preventing and treating inflammatory diseases.

### BACKGROUND

Inflammation is a basic biological response of a body against the invasion of pathogenic microorganisms, which can promote repair of injured cells and tissues, and prevent them from being further injured. However, excessive inflammation can cause injury and necrosis of tissue organs and even systemic organs.

At present, inflammation is a common disease and frequently-occurring disease threatening human health. For treatment of inflammation, people often use glucocorticoid steroidal anti-inflammatory drugs and traditional non-steroidal anti-inflammatory drugs. Even though the existing anti-inflammatory drugs can effectively control infectious inflammation and non-infectious inflammation and effectively eliminate the functional injury disorder caused by inflammation, but if being used for a long term, triggers the function decline of adrenal cortex and other complications. Since the traditional anti-inflammatory drug has the defects of poor selectivity and obvious side effects, it is greatly limited in clinical application. Thus, it is urgent to develop a new types of anti-inflammatory drugs with good curative effects and little side effects.

After taking dexamethasone-based glucocorticoids, about 35% of patients have remarkable adverse reactions, which mostly occur in the application of pharmacological dose, and is closely related to the course of treatment, doses, medication types, usages and administration routes. There are several common adverse reactions: iatrogenic Cushing's syndrome has mental symptoms such as delirium. The nucleotide derivative can be metabolized into products beneficial to a human body after entering the human body, and has significant curative effects on inflammation, and the incidence of adverse reactions is as low as 3%.

Pancreatitis is a common gastrointestinal disease. The incidence rate of acute pancreatitis is 33.74 cases (100 thousand of people each year), the mortality of acute pancreatitis is 1.60 cases (100 thousand of people each year), and it is a more and more common issue that children have acute pancreatitis. Although the existing diagnosis level and treatment level are being constantly improved, the morbidity, recurrence rate and mortality of acute pancreatitis still remain high. In China, the morbidity of AP is in a rising trend year by year. The clinical epidemiological characteristic results of 1316 cases of acute pancreatitis in Guangdong show that from 1986 to 2005, the total number of AP cases is from 0.19% to 0.71% of total number of inpatients in internal and external departments during the corresponding period. In Britain, the morbidity of AP is from 27.6 cases per 100,000 people in 1999 to 36.4 cases per 100,000 people in 2010, with an average annual increase of 2.7%. For treatment of acute pancreatitis, there are no specific treatment regimens at present, main treatment regiments include nutritional support, antibiotic treatment, surgical treatment and integrated traditional Chinese and Western medicine treatment in accordance with Chinese Acute Pancreatitis Diagnosis and Treatment Guidelines (2014) and American Gastroenterology Association Acute Pancreatitis Treatment Guidelines (2013). Therefore, it is necessary to develop drugs with significant anti-pancreatitis effects, especially anti-acute pancreatitis effects.

A liver, as an important organ of the body, undertakes important physiological functions of the body, such as main metabolism, detoxification and secretion. Meanwhile, it is also a lymphoid organ having unique immunological characteristics, and participates in natural immune and adaptive immune responses. If liver dysfunction occurs, various immune diseases are generated. Autoimmune hepatitis is a chronic liver disease which has ambiguous pathogenesis accompanied with obvious autoimmune phenomena and inflammatory necrosis as the main pathological change, this disease is wide in epidemiology, and severely impairs human's health and brings huge economic loss. Autoimmune hepatitis is a chronic inflammatory liver disease, which can cause increase of serum transaminase, has an autoantibody in the process of circulation, has the features of high γ-globulinemia, liver histological characteristic change and response to immunosuppressive therapy, and can lead to liver cirrhosis and liver failure. Therefore, it is extremely necessary to develop drugs having a significant anti-immune hepatitis effect.

Rheumatoid arthritis is a systemic autoimmune disease with chronic destructive arthropathy as a main characteristic, which mainly injurys articular cartilages, bones and joint capsules, and if seriously, can result in outcomes such as joint deformity and function loss. Rheumatoid arthritis lesions are characterized by synovitis and the resulting injurys on articular cartilages and bones, which finally causes joint deformity. Rheumatoid arthritis may occur in all ages, its morbidity is about 1%, peak ages are between 30 and 50, and the female morbidity is more than male morbidity; without regular treatment, many patients may be disabled within 3 years. Treatment methods mainly include general treatment, medicine treatment, surgical operation and other treatments, however, it is unclear at present that treatment of rheumatoid arthritis should select which anti-rheumatic drug to effectively improve the conditions. If rheumatoid arthritis cannot be effectively treated in time, its disability rate is high. The main treatment purpose of rheumatoid arthritis lies in condition control as well as joint function and prognosis improvement. Currently, there is an urgent need to develop effective anti-rheumatoid arthritis drugs.

### SUMMARY

The objective of the present invention is to provide a nucleoside derivative for preventing and treating inflammation and an application thereof in preparation of a drug for preventing and treating inflammatory diseases to improve the curative effect of inflammatory diseases, in order to the defects and disadvantages existing in the existing drugs for preventing and treating inflammation.

The nucleoside derivative for preventing and treating inflammation provided by the present invention is a lead compound based on caffeine, which is obtained by structure modification and optimization. The structure formula of the nucleoside derivative for preventing and treating inflammation is represented by formula (I) ~ formula (IV), or the derivative is a pharmaceutically acceptable salt of a compound of formula (I) ~ formula (IV),

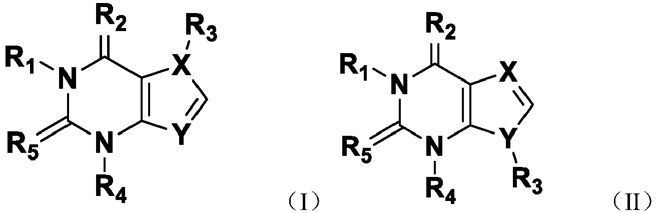

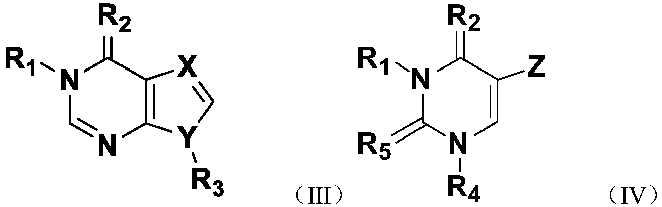

in formula (I) ~ formula (IV), R₁ is H, alkyl, heterocyclyl, alkyl substituted with an oxygen atom, a sulfur atom, a nitrogen atom or halogen, an ester group, amido or amino; R₃ and R₄ are alkyl, heterocyclyl, alkyl substituted with an oxygen atom, a sulfur atom, a nitrogen atom or halogen; R₂ and R₅ are O or S, X and Y are N or C, and Z is halogen.

In the technical solution of the above nucleoside derivative for preventing and treating inflammation, in formula (I) ~ formula (IV), R₁ is preferably H, (C₁-C₁₈) alkyl, (C₃-C₁₂) heterocyclyl, (C₁-C₁₈) alkyl substituted with an oxygen atom, a sulfur atom, a nitrogen atom or halogen, ester group, amido or amino; R₃ and R₄ are preferably (C₁-C₃) alkyl.

Further preferably, R₁ is H, (C₁-C₃) alkyl, (C₃-C₁₂) heterocyclyl, (C₁-C₃) alkyl substituted with an oxygen atom, a sulfur atom, a nitrogen atom or halogen, or is a group equivalent to -A₁OC(O)A₂, -A₁NHA₂ and -A₁NHCOA₂, wherein A₁ and A₂ are (C₁-C₃) alkyl and (C₁-C₃) alkyl substituted with an oxygen atom, a sulfur atom, a nitrogen atom or halogen.

More further preferably, R₁ is H, (C₁-C₃) alkyl, or is a group equivalent to -A₁OC(O)A₂, -A₁NHA₂ and -A₁NHCOA₂, wherein A₁ and A₂ are (C₁-C₃) alkyl. More further preferably, in formula (I) ~ formula (IV), R₁ is (C₁-C₃) alkyl, or is a group equivalent to -A₁OC(O)A₂, -A₁NHA₂ and -A₁NHCOA₂, wherein A₁ and A₂ are (C₁-C₃) alkyl; in formula (I) ~ formula (IV), R₁ is H.

More further preferably, the above nucleoside derivative for preventing and treating inflammation is any one of the following compounds 1~19, or a pharmaceutically acceptable salt of any one of the following compounds 1~19:

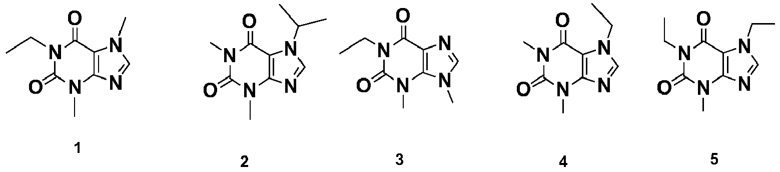

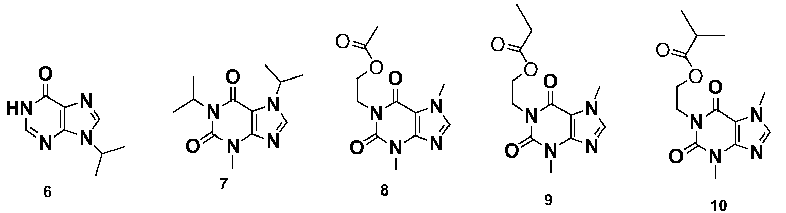

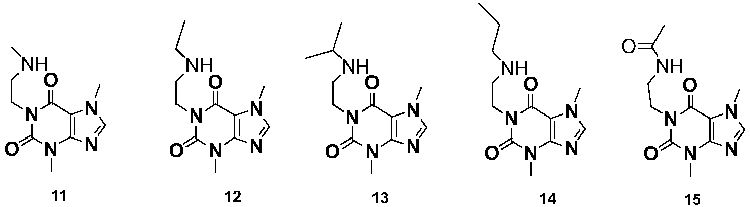

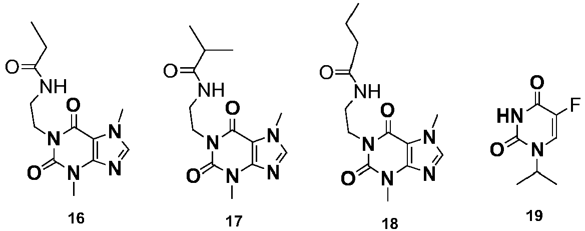

In the technical solution of the above nucleoside derivative for preventing and treating inflammation, pharmaceutically acceptable salts of compounds of formula (I)~formula (II) and a pharmaceutically acceptable salt of any one of compounds 1~19 are addition salts formed by compounds of formula (I) ~ formula (II) and any one of compounds 1~19 in combination with hydrochloric acid, hydrobromic acid, sulfuric acid, carbonic acid, citric acid, succinic acid, tartaric acid, phosphoric acid, lactic acid, pyruvic acid, acetic acid, maleic acid, methanesulfonic acid, benzenesulfonic acid, p-methylbenzenesulfonic acid or ferulic acid.

The nucleoside derivative for preventing and treating inflammation provided by the present invention can be combined with a pharmaceutically acceptable carrier to jointly exert the activity of preventing and treating inflammation. The nucleoside derivative for preventing and treating inflammation provided by the present invention can be prepared into oral formulations, injection formulations and the like by using a conventional drug method.

The present invention also provides an application of the above nucleoside derivative in preparation of a drug for preventing and treating inflammatory diseases. Further, the inflammatory diseases comprise pancreatitis, hepatitis, arthritis, and related complications of pancreatitis, hepatitis and arthritis. More further, the pancreatitis comprises acute pancreatitis, the hepatitis comprises immune hepatitis, and the arthritis comprises rheumatoid arthritis. In the present invention, animal tests verify that the above nucleoside derivative for preventing and treating inflammation can obviously improve conditions of pancreatitis, hepatitis, arthritis and the like, improve organ injury and inflammatory indexes, and has a better effect than that of positive control drug indometacin.

Compared with the existing prior art, the technical solution of the present invention has the following beneficial effects:

Through experiments, it is verified that the nucleoside derivative for preventing and treating inflammation provided by the present invention can obviously ameliorate the conditions of pancreatitis, hepatitis, arthritis and the like, ameliorate organ injury and inflammatory indexes, and has a better effect than that of positive control drug indomethacin. Moreover, through experiments, it is confirmed that compared with the traditional anti-inflammatory drugs aspirin, ibuprofen, indomethacin, phenylbutazone, diclofenac, piroxicam and glucocorticoid, the nucleoside derivative for preventing and treating inflammation provided by the present invention has the advantages of significantly less side effects. In addition, the nucleoside derivative for preventing and treating inflammation provided by the present invention has simple structure and synthesis process, is suitable for industrial production, can reduce production cost and has the advantage of high economy.

### DESCRIPTION OF THE EMBODIMENTS

Next, the nucleoside derivative for preventing and treating inflammation provided the present invention and application of the derivative in preparation of a drug for preventing and treating inflammatory diseases will be further described through embodiments, it is necessary to point out that the following embodiments are only for further illustrating the present invention, and cannot be considered as limiting the protective scope of the present invention, those skilled in the art make some non-essential improvements and adjustments according to the above inventive contents, which still belong to the protective scope of the present invention.

### Example 1

In this example, compounds 1~19 were synthesized.

### 1. Synthesis of compound 1: 1-ethyl-3-7-dimethylxanthine

540 mg (3 mmol) of theobromine and 720 mg (30 mmol) of NaH were dissolved into 80 ml of DMF, 2 mL (24.7 mmol) of ethane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain 495 mg (2.5 mmol) of compound 1 with a yield of 85%.

The compound 1 was detected via nuclear magnetic hydrogen spectroscopy, nuclear magnetic carbon spectroscopy and mass spectrometry. The results are as follows: ¹H NMR (400 MHz, DMSO) δ 8.00 (s, 1H), 3.92 - 3.85 (m, 5H), 3.40 (s, 3H), 1.11 (t, *J* = 7.0 Hz, 3H).
¹³ C NMR (101 MHz, DMSO) δ 154.09 (s), 150.54 (s), 148.08 (s), 142.75 (s), 106.56 (s), 35.44 (s), 33.05 (s), 29.20 (s), 13.02 (s).
HRMS (TOF MS ES+) for C₉H₁₂N₄O₂Na⁺ (M+Na⁺) calcd 231.0858, found 231.0856.

### 2. Synthesis of compound 2: 1-isopropyl-3,7-dimethylxanthine

1.08 g (6 mmol) of theophylline and 1.8 mg (75 mmol) of NaH were dissolved into 100 ml of DMF, 10 mL (100 mmol) of isopropane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain 1.83 g (5.3 mmol) of compound 2 with a yield of 88%.

The compound 2 was detected via nuclear magnetic hydrogen spectroscopy, nuclear magnetic carbon spectroscopy and mass spectrometry. The results are as follows: ¹H NMR (600 MHz, DMSO) δ 8.19 (s, 1H), 4.91 (hept, *J* = 6.5 Hz, 1H), 3.42 (s, 3H), 3.23 (s, 3H), 1.50 (d, *J* = 6.7 Hz, 6H).
¹³ C NMR (151 MHz, DMSO) δ 154.69 (s), 151.36 (s), 149.16 (s), 140.57 (s), 106.14 (s), 49.88 (s), 29.85 (s), 28.13 (s), 22.90 (s).
HRMS (TOF MS ES+) for C₁₀H₁₄N₄O₂Na⁺ (M+Na⁺) calcd 245.1009, found 245.1013.

### 3. Synthesis of compound 3: 1-ethyl-3,9-dimethylxanthine

3 mmol of 3, 9-dimethylxanthine and 720 mg (30 mmol) of NaH were dissolved into 80 ml of DMF, 2 mL (24.7 mmol) of ethane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 3 with a yield of 89%.

The compound 3 was detected via nuclear magnetic hydrogen spectroscopy. The results are as follows: ¹H NMR (400 MHz, Methanol-*d*₄) δ 7.88(s, 1H), 3.92(q, 2H), 3.72(s, 3H), 3.28(s, 3H), 1.14(t, 3H).

### 4. Synthesis of compound 4: 1,3-dimethyl-7-ethylxanthine

3 mmol of 1,3-dimethylxanthine and 720 mg (30 mmol) of NaH were dissolved into 80 ml of DMF, 2 mL (24.7 mmol) of ethane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 4 with a yield of 86%.

The compound 4 was detected via nuclear magnetic hydrogen spectroscopy, nuclear magnetic carbon spectroscopy and mass spectrometry. The results are as follows: ¹H NMR (400 MHz, DMSO) δ 8.08 (s, 1H), 4.26 (q, *J* = 7.2 Hz, 2H), 3.41 (s, 3H), 3.22 (s, 3H), 1.39 (t, J = 7.2 Hz, 3H).
¹³C NMR (101 MHz, DMSO) δ154.17, 150.94, 148.37, 141.78, 105.75, 41.44, 29.32, 27.47, 16.24.
HRMS (TOF MS ES+) for C₉H₁₂N₄O₂Na⁺ (M+Na⁺) calcd 231.0858, found 231.0858.

### 5. Synthesis of compound 5: 1,7-diethyl-3-methylxanthine

3 mmol of 3-methylxanthine and 720 mg (30 mmol) of NaH were dissolved into 80 ml of DMF, 4 mL (49.4 mmol) of ethane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 5 with a yield of 92%.

The compound 5 was detected via nuclear magnetic hydrogen spectroscopy, nuclear magnetic carbon spectroscopy and mass spectrometry. The results are as follows: ¹H NMR (400 MHz, DMSO) δ 8.09 (s, 1H), 4.28 (q, *J* = 7.2 Hz, 2H), 3.91 (q, *J* = 7.0 Hz, 2H), 3.42 (s, 3H), 1.41 (t, *J* = 7.2 Hz, 3H), 1.13 (t, *J* = 7.0 Hz, 3H) ppm.
¹³C NMR (101 MHz, DMSO) δ 153.80 (s), 150.52 (s), 148.42 (s), 141.79 (s), 105.83 (s), 41.43 (s), 35.51 (s), 29.22 (s), 16.19 (s), 13.01 (s) ppm.
HRMS (TOF MS ES+) for C₁₀H₁₄N₄O₂Na⁺ (M+Na⁺) calcd 245.1014,found 245.1016.

### 6. Synthesis of compound 6: 9-isopropyl hypoxanthine

6 mmol of hypoxanthine and 1.8 g (75 mmol) of NaH were dissolved into 100 ml of DMF, 10 mL (100 mmol) of isopropane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 6 with a yield of 75%.

The compound 6 was detected via nuclear magnetic hydrogen spectroscopy. The results are as follows: ¹H NMR (500 MHz, Chloroform-d) δ 8.28 (d, J = 7.6 Hz, 1H), 7.64 (s, 1H), 4.73 (p, J = 6.3 Hz, 1H), 1.52 (d, J = 6.8 Hz, 6H).

### 7. Synthesis of compound 7: 1,7-diisopropyl-3-methylxanthine

6 mmol of 3-methylxanthine and 1.8 g (75 mmol) of NaH were dissolved into 100 ml of DMF, 10 mL (100 mmol) of isopropane iodide was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 7 with a yield of 87%.

The compound 7 was detected via nuclear magnetic hydrogen spectroscopy. The results are as follows: ¹H NMR (500 MHz, Chloroform-d) δ 8.33 (s, 1H), 4.92 (hept, J = 6.2 Hz, 1H), 4.32 (hept, J = 6.2 Hz, 1H), 3.21 (s, 2H), 1.51 (d, J = 6.1 Hz, 5H), 1.31 (d, J = 6.1 Hz, 5H).

### 8. Synthesis of compound 8: 1-(acetic acid-2-ethyl)-3,7-dimethylxanthine

540mg (3 mmol) of theobromine and 720 mg (30 mmol) of NaH were dissolved into 80 ml of DMF, 4 mmol of 2-iodoethanol was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-ethylhydroxy-3-7-dimethylxanthine with a yield of 94%.

3 mmol of 1-ethylhydroxy-3-7-dimethylxanthine, 3 mmol of acetic anhydride and 0.3 mmol of NaOH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 8 with a yield of 82%.

The compound 8 was detected via nuclear magnetic hydrogen spectroscopy. The results are as follows: ¹H NMR (400 MHz, DMSO) δ 8.02 (s, 1H), 4.20 (t, *J* = 5.5 Hz, 2H), 4.11 (t, *J* = 5.6 Hz, 2H), 3.88 (s, 3H), 3.41 (s, 3H), 1.95 (s, 3H).¹³C NMR (101 MHz, DMSO) δ 170.74 (s), 154.86 (s), 151.39 (s), 148.82 (s), 143.52 (s), 107.01 (s), 61.34 (s), 33.63 (s), 29.83

(s), 21.09 (s).

### 9. Synthesis of compound 9: 1-(propionic acid -2-ethyl)-3,7-dimethylxanthine

540mg (3 mmol) of theobromine and 720 mg (30 mmol) of NaH were dissolved into 80 ml of DMF, 4 mmol of 2-iodoethanol was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-ethylhydroxy-3-7-dimethylxanthine with a yield of 94%.

3 mmol of 1-ethylhydroxy-3-7-dimethylxanthine, 3 mmol of propionic anhydride and 0.3 mmol of NaOH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 9 with a yield of 80%.

The compound 9 was detected via nuclear magnetic hydrogen spectroscopy. The results are as follows: ¹H NMR (500 MHz, Chloroform-d) δ 7.54 (s, 1H), 4.34 (t, J = 6.3 Hz, 1H), 3.76 (s, 1H), 3.54 (t, J = 6.32 Hz, 1H), 3.54 (s, 1H), 2.34 (q, J = 7.4 Hz, 1H), 1.43 (t, J = 7.9 Hz, 1H).

### 10. Synthesis of compound 10: 1-(isopropyl acid-2-ethyl)-3,7-dimethylxanthine

540mg (3 mmol) of theobromine and 720 mg (30 mmol) of NaH were dissolved into 80 ml of DMF, 4 mmol of 2-iodoethanol was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-ethylhydroxy-3-7-dimethylxanthine with a yield of 94%.

3 mmol of 1-ethylhydroxy-3-7-dimethylxanthine, 3 mmol of isopropyl anhydride and 0.3 mmol of NaOH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 10 with a yield of 81%.

The compound 10 was detected via nuclear magnetic hydrogen spectroscopy. The results are as follows: ¹H NMR (500 MHz, Chloroform-d) δ 7.62 (s, 0H), 4.43 (t, J = 6.2 Hz, 1H), 3.46 (s, 1H), 3.48 (t, J = 6.5 Hz, 1H), 3.43 (s, 1H), 2.53 (hept, J = 7.2 Hz, 0H), 1.25 (d, J = 7.4 Hz, 3H).

### 11. Synthesis of compound 11: 1-(2-methylaminoethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-(2-iodoethyl)-3-7-dimethylxanthine with a yield of 89%.

3 mmol of 1-(2-iodoethyl)-3-7-dimethylxanthine, 3 mmol of methylamine and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 11 with a yield of 80%.

The compound 11 was detected via nuclear magnetic hydrogen spectroscopy. The results are as follows: ¹H NMR (500 MHz, Chloroform-*d*) δ 7.54 (s, 1H), 4.33 (t, *J* = 5.1 Hz, 2H), 3.77 (s, 2H), 3.32 (s, 2H), 2.84 (td, *J* = 5.4, 3.5 Hz, 2H), 2.56 (d, *J* = 4.8 Hz, 3H), 2.34 (qt, *J* = 4.3, 3.5 Hz, 1H).

### 12. Synthesis of compound 12: 1-(2-ethylaminoethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-(2-iodoethyl)-3-7-dimethylxanthine with a yield of 89%.

3 mmol of 1-(2-iodoethyl)-3-7-dimethylxanthine, 3 mmol of ethylamine and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 12 with a yield of 78%.

The compound 12 was detected via nuclear magnetic hydrogen spectroscopy. The results are as follows: ¹H NMR (500 MHz, Chloroform-*d*) δ 7.54 (s, 1H), 4.01 - 3.72 (m, 4H), 3.41 (s, 2H), 2.83 (td, *J* = 5.2, 4.0 Hz, 2H), 2.76 (p, *J* = 4.3 Hz, 1H), 2.62 (qd, *J* = 7.2, 4.2 Hz, 2H), 1.32 (t, *J* = 7.3 Hz, 3H).

### 13. Synthesis of compound 13: 1-(2-isopropylaminoethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 1-(2-iodoethyl)-3-7-dimethylxanthine with a yield of 89%.

3 mmol of 1-(2-iodoethyl)-3-7-dimethylxanthine, 3 mmol of isopropylamine and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 13 with a yield of 79%.

The compound 13 was detected via nuclear magnetic hydrogen spectroscopy. The results are as follows: ¹H NMR (500 MHz, Chloroform-*d*) δ 7.52 (s, 1H), 4.23 (t, *J* = 5.3 Hz, 2H), 3.84 (s, 2H), 3.41 (s, 2H), 3.03 (td, *J* = 5.5, 3.5 Hz, 2H), 2.81 (dp, *J* = 7.4, 6.4 Hz, 1H), 2.35 (dt, *J* = 7.3, 3.7 Hz, 1H), 1.13 (d, *J* = 6.1 Hz, 6H).

### 14. Synthesis of compound 14: 1-(2-propylaminoethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 1-(2-iodoethyl)-3-7-dimethylxanthine with a yield of 89%.

3 mmol of 1-(2-iodoethyl)-3-7-dimethylxanthine, 3 mmol of propylamine and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 14 with a yield of 78%.

The compound 14 was detected via nuclear magnetic hydrogen spectroscopy. The results are as follows: ¹H NMR (500 MHz, Chloroform-*d*) δ 7.63 (s, 1H), 4.03 (t, *J* = 5.4 Hz, 2H), 3.92 (s, 2H), 3.48 (s, 2H), 2.82 (td, *J* = 5.4, 4.2 Hz, 2H), 2.76 (p, *J* = 4.3 Hz, 1H), 2.86 (td, *J* = 6.2, 4.2 Hz, 2H), 1.54 (qt, *J* = 7.4, 6.1 Hz, 2H), 0.92 (t, *J* = 7.4 Hz, 3H).

### 15. Synthesis of compound 15: 1-(2-acetylethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether:
CH₃CH₂OCOCH₃ = 3:1 (V/V) as a mobile phase to obtain the compound 1-(2-iodoethyl)-3-7-dimethylxanthine with a yield of 89%.

3 mmol of 1-(2-iodoethyl)-3-7-dimethylxanthine, 3 mmol of acetamide and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 15 with a yield of 79%.

The compound 15 was detected via nuclear magnetic hydrogen spectroscopy. The results are as follows: ¹H NMR (500 MHz, Chloroform-*d*) δ 7.54 - 7.65 (m, 1H), 4.12 (t, *J* = 5.4 Hz, 1H), 3.65 (s, 1H), 3.65 (td, *J* = 5.4, 4.4 Hz, 1H), 3.48 (s, 1H), 1.87 (s, 1H).

### 16. Synthesis of compound 16: 1-(2-propionyl ethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 1-(2-iodoethyl)-3-7-dimethylxanthine with a yield of 89%.

3 mmol of 1-(2-iodoethyl)-3-7-dimethylxanthine, 3 mmol of propanamide and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 16 with a yield of 76%.

The compound 16 was detected via nuclear magnetic hydrogen spectroscopy. The results are as follows: ¹H NMR (500 MHz, Chloroform-*d*) δ 7.54 (t, *J =* 4.1 Hz, 1H), 7.65 (s, 1H), 4.21 (t, *J* = 5.3 Hz, 2H), 3.76 (s, 2H), 3.65 (td, *J* = 5.4, 4.4 Hz, 2H), 3.54 (s, 2H), 2.24 (q, *J* = 7.9 Hz, 2H), 1.25 (t, *J* = 7.9 Hz, 3H).

### 17. Synthesis of compound 17: 1-(2-isopropionyl ethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-(2-iodoethyl)-3-7-dimethylxanthine with a yield of 89%.

3 mmol of 1-(2-iodoethyl)-3-7-dimethylxanthine, 3 mmol of isopropanamide and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 17 with a yield of 79%.

The compound 17 was detected via nuclear magnetic hydrogen spectroscopy. The results are as follows: ¹H NMR (500 MHz, Chloroform-d) δ 7.45 (s, 1H), 7.65 (t, *J =* 4.4 Hz, 1H), 4.43 (t, *J=* 5.3 Hz, 2H), 3.65 (s, 2H), 3.65 (td, *J=* 5.5, 4.1 Hz, 2H), 3.43 (s, 2H), 2.35 (hept, *J* = 7.2 Hz, 1H), 1.32 (d, *J* = 7.3 Hz, 5H).

### 18. Synthesis of compound 18: 1-(2-butyrylethyl)-3,7-dimethylxanthine

540mg (3 mmol) of theobromine and 720 mg (30 mmol) of NaH were dissolved into 80 ml of DMF, 4 mmol of 1,2-diiodoethane was dropwise added under the stirring, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain compound 1-(2-iodoethyl)-3-7-dimethylxanthine with a yield of 89%.

3 mmol of 1-(2-iodoethyl)-3-7-dimethylxanthine, 3 mmol of butyrylamide and 0.3 mmol of NaH were dissolved into 80 ml of DMF, the reaction was quenched with water after 4 hours of reaction, and the reaction product was subjected to spin drying under reduced pressure, and then loaded to a silica gel column with petroleum ether: CH₃CH₂OCOCH₃ = 3:1 (V/V) as a mobile phase to obtain the compound 18 with a yield of 79%.

The compound 18 was detected via nuclear magnetic hydrogen spectroscopy. The results are as follows: ¹H NMR (500 MHz, Chloroform-*d*) δ 7.73 - 7.62 (m, 1H), 4.13 (t, *J* = 5.5 Hz, 1H), 3.76 (s, 1H), 3.46 (td, *J* = 5.4, 4.2 Hz, 1H), 3.43 (s, 1H), 2.43 - 2.13 (m, 1H), 1.54 (h, *J* = 7.4 Hz, 1H), 0.98 (t, *J* = 7.4 Hz, 1H).

### 19. Synthesis of compound 19: 3-isopropyl-5-fluorouracil

6 mmol of 5-fluorouracil and 1.8 g (75 mmol) of NaH were dissolved into 100 ml of DMF, 1 mL (10 mmol) of iodoisopropane was dropwise added under the stirring, the reaction was quenched with water after 4 hours of reaction, and the reaction product was subjected to spin drying under reduced pressure, and then loaded to a silica gel column with petroleum ether:
CH₃CH₂OCOCH₃ = 3:1 (V/V) as a mobile phase to obtain the compound 19 with a yield of 87%.

The compound 19 was detected via nuclear magnetic hydrogen spectroscopy. The results are as follows: ¹H NMR (400 MHz, DMSO) δ 11.72 (s, 1H), 8.13 (d, *J* = 7.3 Hz, 1H), 4.64 (dt, *J* = 13.6, 6.8 Hz, 1H), 1.24 (d, *J* = 6.8 Hz, 6H).

### Example 2

In this example, the improvement effect of compounds 1~19 prepared in example 1 on pancreatits was measured.

### 1. Test method

### (1) Animals: adult and healthy male Blab/c mice, 25~30g.

### (2) Grouping

Blab/c mice were divided into blank control group, model control group, caffeine treatment group, and compound 1 ~ compound 19 treatment group, 10 mice/group.

### (3) Construction of pancreatits model

A model was made with sodium taurocholate. Prior to the experiment, the mice were fasted for 12 hours with water, then operated in a sterile environment, and subsequently underwent intraperitoneal injection with 3.5% sodium pentobarbital. After anesthesia, the surgical site was sterilized with an alcohol cotton ball. The sterile gauze was placed on the surgical site. A laparotomy was made at the width of two fingers under the xiphoid process. After seeing the liver, an incision was made, the cotton swab soaked in normal saline was stretched into the enterocoelia, the duodenum was sought on the back of the liver, turned out and placed on the sterile gauze, the pancreaticobiliary duct and duodenal papilla were sought, and the needle of 1 mL syringe was used to pierce a hole on the along the direction of the nipple, a 24G indwelling needle tube was inserted into the pancreatic duct along the nipple, and the common bile duct below the liver was closed with an arterial vascular clip, with a dose of 0.1 mL/100 g and 0.1 mL/min inject sodium taurocholate with a concentration of 3.8% at a flow rate. During this process, pay attention to adding saline to the exposed pancreas and duodenum to keep it moist. After the injection, the tube was slowly taken out and the bile duct was continued to be closed. The main tube was removed from the vascular artery clip 3 minutes later, the duodenum was also accommodated, and the abdominal cavity was sutured in double layers. The blank control group was injected with equal volume of isotonic saline.

### (4) Administration

For caffeine treatment group and compound 1 ~ compound 19 treatment group, caffeine and compound 1 ~ compound 19 were injected into caudal vein at the dose of 17.5 mg/kg 1 h, 3 h and 6 h after pancreatitis models were respectively constructed.

The blank control group and the model control group were given equal volume of isotonic saline.

### (5) Index measurement

After 24 hours, pancreatic tissue and blood samples were taken, and the pancreatic histopathological injury score and the level of pancreatic amylase in serum were evaluated.

### 2. Results

The pancreatic histopathological injury score and pancreatic amylase level of mice in each group are shown in Table 1. It can be seen from Table 1 that compounds 1 ~ 19 provided by the present invention can effectively reduce the pancreatic amylase level of sodium taurocholate-induced pancreatitis model so as to alleviate the injury of pancreas.

**Table 1 Pancreatic histopathological injury score and pancreatic amylase level of mice in each group (n = 10)**

| Group | Pancreatic histopathological injury score | Amylopsin (KU/L) | Group | Pancreatic histopathological injury score | Amylopsin (KU/L) |
|---|---|---|---|---|---|
| Blank control group | 0.3 ± 0.2 | 2± 3 | Compound 9 treatment group | 0.5 ± 0.4 | 5 ± 3 |
| Model control group | 3.8 ± 0.3 | 24 ± 3 | Compound 10 treatment group | 0.6 ± 0.4 | 7 ± 5 |
| Caffeine treatment group | 2.6 ± 0.4 | 16 ± 2 | Compound 11 treatment group | 0.5 ± 0.2 | 5 ± 3 |
| Compound 1 treatment group | 0.4 ± 0.3 | 3 ± 1 | Compound 12 treatment group | 0.4 ± 0.5 | 4 ± 2 |
| Compound 2 treatment group | 0.6 ± 0.2 | 4 ± 2 | Compound 13 treatment group | 1.8 ± 0.2 | 6 ± 2 |
| Compound 3 treatment group | 0.5 ± 0.2 | 3 ± 2 | Compound 14 treatment group | 1.9 ± 0.4 | 5 ± 3 |
| Compound 4 treatment group | 1.4 ± 0.4 | 11 ::I:: 2 | Compound 15 treatment group | 1.6 ± 0.4 | 5 ± 4 |
| Compound 5 treatment group | 0.4 ± 0.2 | 3 ± 1 | Compound 16 treatment group | 1.8 ± 0.3 | 5 ± 2 |
| Compound 6 treatment group | 0.4 ± 0.1 | 2 ± 1 | Compound 17 treatment group | 1.5 ± 0.4 | 10 ± 3 |
| Compound 7 treatment group | 0.5 ± 0.2 | 4 ± 2 | Compound 18 treatment group | 1.3 ± 0.4 | 9 ± 2 |
| Compound 8 treatment group | 0.8 ± 0.4 | 5 ± 2 | Compound 19 treatment group | 1.0 ± 0.3 | 8 ± 2 |

### Example 3

In this example, the improvement effect of compounds 1~19 prepared in example 1 on arthritic was measured.

### 1. Test method

(1) Animals: SD rats, four rates in one cage, were fed under the conditions of constant temperature (25 ± 2 °C) and illumination control (12 h day/night circulation). The animals were fed with standard feed, and fed for at least 7 days to adapt environment before the experiment.

### (2) Grouping

SD rates were divided into blank control group, model control group, caffeine treatment group, indometacin treatment group and compound 1~compound 19 treatment group, 10 mice/group.

(3) SD rats were fasted for 12 hours before operation, and weighed and recorded prior to operation. The blank control group was not treated any more.

SD rats in model control group, caffeine treatment group, indomethacin treatment group and compound 1 - compound 19 treatment group were intraperitoneally injected with 10% chloral hydrate solution at the dose of 3.5 ml/kg. After anesthesia, they were fixed in the supine position. 0.1 ml of 1 g/L sodium iodoacetate solution was injected into the knee joint. From the fifth day, the animals were driven to run for 30 minutes every day, and they were allowed to move freely in the cage the rest of the time. After modeling, indomethacin and compounds 1 ~ 19 were given by gavage every day at the dose of 100 mg/kg/d. The blank control group and the model control group were given equal volume of isotonic saline.

### (4) Index measurement

At the 4th week after operation, the articular fluid of rats in each group was extracted to measure the content of MMP-13. The rats were killed, and the subchondral bone plate of the inner condyle of the tibia of the right knee was cut and processed into specimens, and pathological scores were made.

### 2. Results

The content of MMP-13 measured in the joint fluid and the score of joint pathological tissue of rats in each group are shown in Table 2. It can be seen from Table 2 that compared with the model control group, compound 1 ~ compound 19 treatment group can effectively reduce the level of MMP-13 in the arthritis model and reduce joint injury. Meanwhile, compared with the positive control drug indomethacin treatment group, the compound 1 ~ compound 19 treatment group has a significantly better improvement effect on arthritis.

**Table 2 MMP-13 contents and joint pathological tissue scores in joint fluid of rats in each group (n = 10)**

| Group | Pathological tissue score | MMP-13 content (mg/L) | Group | Pathological tissue score | MMP-13 content (mg/L) |
|---|---|---|---|---|---|
| Blank control group | 0.1± 0.2 | 81± 7 | Compound 9 treatment group | 0.6 ± 0.2 | 97 ± 7 |
| Model control group | 3.2 ± 0.3 | 256 ± 24 | Compound 10 treatment group | 0.7 ± 0.4 | 91 ± 8 |
| Caffeine treatment group | 2.6 ± 0.4 | 59 ± 8 | Compound 11 treatment group | 0.3 ± 0.3 | 86 ± 5 |
| Indometacin treatment group | 2.1 ± 0.3 | 195 ± 8 | Compound 12 treatment group | 0.3 ± 0.4 | 83 ± 8 |
| Compound 1 treatment group | 0.2 ± 0.3 | 86 ± 4 | Compound 13 treatment group | 0.3 ± 0.5 | 98 ± 5 |
| Compound 2 treatment group | 0.3 ± 0.4 | 92 ± 8 | Compound 14 treatment group | 0.7 ± 0.3 | 93 ± 7 |
| Compound 3 treatment group | 0.6 ± 0.3 | 97 ± 4 | Compound 15 treatment group | 0.6 ± 0.5 | 91 ± 7 |
| Compound 4 treatment group | 0.6 ± 0.4 | 102 ± 8 | Compound 16 treatment group | 0.4 ± 0.3 | 93 ± 9 |
| Compound 5 treatment group | 0.7 ± 0.3 | 99 ± 5 | Compound 17 treatment group | 0.5 ± 0.5 | 99 ± 6 |
| Compound 6 treatment group | 0.3 ± 0.4 | 90 ± 8 | Compound 18 treatment group | 0.5 ± 0.3 | 91 ± 8 |
| Compound 7 treatment group | 0.3 ± 0.3 | 84 ± 5 | Compound 19 treatment group | 0.3 ± 0.2 | 89 ± 6 |
| Compound 8 treatment group | 0.4 ± 0.5 | 92 ± 8 | | | |

### Example 4

In this example, the improvement effect of compounds 1~19 prepared in example 1 on pepatitis was measured.

### 1. Test method

### (1) Animals: adult and healthy male Blab/c mice, 25~30g.

### (2) Grouping

Blab/c mice were adaptively fed for one week and then divided into blank control group, model control group, caffeine treatment group, and compound 1~compound 19 treatment group at random, 10 mice/group.

(3) Blab/c mice in model control group, caffeine treatment group, indomethacin treatment group and compound 1 ~ compound 19 treatment group were injected with concanavalin A (ConA) solution through caudal vein to form a mouse hepatitis model. The blank control group was injected with equal volume of isotonic saline.

After modeling, indomethacin and compounds 1~19 were given by gavage every day at the dose of 50 mg/kg. The blank control group and the model control group were given equal volume of isotonic saline.

### (4) Index measurement

Eyeball blood and liver tissue were taken at 6 h, 12 h, 24 h and 48 h after administration. The levels of serum alanine aminotransferase (ALT) and aspartate aminotransferase (AST) were measured.

### 2. Results

### (1) Serum alanine aminotransferase and aspartate aminotransferase levels

After 6 h, 12 h, 24 h and 48 h of administration, the levels of serum alanine aminotransferase and aspartate aminotransferase of mice in each group are shown in Table 3. It can be seen from Table 3 that compared with the model control group, the compound 1 ~ 19 treatment group can significantly reduce the levels of serum alanine aminotransferase and aspartate aminotransferase and reduce liver injury. Meanwhile, compared with the positive control drug indomethacin treatment group, the compound 1 - compound 19 treatment group has a significantly better improvement effect on hepatitis.

**Table 3 Serum alanine aminotransferase and aspartate aminotransferase levels of mice in each group (n = 10)**

| Group | Serum alanine aminotransferase (ALT, U/L) | | | | Aspartate aminotransferase (AST, U/L) | | | |
|---|---|---|---|---|---|---|---|---|
| | 6 (h) | 12 (h) | 24 (h) | 48 (h) | 6 (h) | 12 (h) | 24 (h) | 48 (h) |
| Blank control group | 12 ± 3 | 11 ± 3 | 13 ± 2 | 12 ± 3 | 25 ± 4 | 22 ± 4 | 25 ± 5 | 23 ± 4 |
| Model control group | 890± 73 | 1832 ± 173 | 3565± 363 | 1987± 193 | 320 ± 72 | 1035± 123 | 1534± 143 | 1243±1 21 |
| Caffeine treatment group | 590± 54 | 1456± 132 | 2632± 321 | 1432± 152 | 260 ± 31 | 860± 91 | 1234± 133 | 943 ± 91 |
| Indometacin treatment group | 465± 43 | 1232 ± 113 | 2165± 203 | 1287± 133 | 220 ± 19 | 780 ± 82 | 1021 ± 103 | 870 ± 91 |
| Compound 1 treatment group | 56 ± 8 | 97 ± 8 | 132 ± 12 | 100 ± 12 | 43 ± 7 | 82 ± 8 | 104 ± 8 | 89 ± 9 |
| Compound 2 treatment group | 59 ± 8 | 98 ± 6 | 142 ± 12 | 112 ± 13 | 53 ± 7 | 89 ± 8 | 112 ± 10 | 99 ± 9 |
| Compound 3 treatment group | 67 ± 8 | 102 ± 8 | 152 ± 14 | 113 ± 12 | 59 ± 7 | 96 ± 8 | 114 ± 11 | 103 ± 9 |
| Compound 4 treatment group | 79 ± 8 | 118 ± 9 | 142 ± 12 | 105 ± 13 | 65 ± 7 | 91 ± 8 | 103 ± 9 | 96 ± 9 |
| Compound 5 treatment group | 73 ± 8 | 109 ± 8 | 136 ± 12 | 105 ± 12 | 71 ± 7 | 93 ± 8 | 101 ± 8 | 91 ± 9 |
| Compound 6 treatment group | 56 ± 5 | 85 ± 6 | 136 ± 16 | 103 ± 13 | 49 ± 7 | 86 ± 8 | 103 ± 9 | 84 ± 8 |
| Compound 7 treatment group | 51 ± 8 | 93 ± 8 | 131 ± 12 | 95 ± 12 | 41 ± 7 | 78 ± 8 | 101 ± 8 | 84 ± 7 |
| Compound 8 treatment group | 67 ± 8 | 110 ± 13 | 172 ± 16 | 132 ± 13 | 68 ± 7 | 95 ± 8 | 122 ± 12 | 98 ± 9 |
| Compound 9 treatment group | 78 ± 8 | 110 ± 13 | 175 ± 18 | 123 ± 12 | 78 ± 7 | 123 ± 8 | 142 ± 13 | 131 ± 14 |
| Compound 10 treatment group | 83 ± 8 | 132 ± 13 | 186 ± 19 | 154 ± 17 | 87 ± 9 | 167 ± 19 | 189 ± 15 | 176 ± 17 |
| Compound 11 treatment group | 54 ± 6 | 92 ± 9 | 142 ± 14 | 110 ± 13 | 48 ± 7 | 89 ± 8 | 112 ± 12 | 95 ± 9 |
| Compound 12 treatment group | 76 ± 8 | 113 ± 12 | 162 ± 16 | 121 ± 13 | 58 ± 7 | 93 ± 9 | 121 ± 19 | 102 ± 9 |
| Compound 13 treatment group | 87 ± 9 | 121 ± 13 | 172 ± 17 | 143 ± 12 | 63 ± 7 | 102 ± 12 | 132 ± 13 | 93 ± 9 |
| Compound 14 treatment group | 56 ± 8 | 134 ± 15 | 193 ± 12 | 132 ± 13 | 65 ± 7 | 93 ± 12 | 152 ± 15 | 119 ± 12 |
| Compound 15 treatment group | 65 ± 8 | 171 ± 15 | 218 ± 26 | 132 ± 12 | 65 ± 7 | 118 ± 18 | 198 ± 18 | 129 ± 9 |
| Compound 16 treatment group | 61 ± 8 | 101 ± 16 | 132 ± 12 | 104 ± 13 | 59 ± 7 | 93 ± 11 | 121 ± 12 | 103 ± 9 |
| Compound 17 treatment group | 79 ± 8 | 145 ± 13 | 163 ± 16 | 142 ± 15 | 65 ± 5 | 102 ±12 | 119 ± 12 | 107 ± 11 |
| Compound 18 treatment group | 67 ± 8 | 106 ± 11 | 157 ± 15 | 121 ± 13 | 54 ± 7 | 98 ± 8 | 131 ± 8 | 117 ± 9 |
| Compound 19 treatment group | 55 ± 6 | 87 ± 6 | 139 ± 12 | 111 ± 13 | 57 ± 7 | 92 ± 8 | 117 ± 9 | 101 ± 9 |

### Example 5

In this example, the toxicities of compounds 1-19 prepared in example 1 were analyzed.

C57 mice were given compounds 1-19 at a dose of 100 mg/kg/D for 180 consecutive days by gavage. One hour after the last administration of the drug, the mice were anesthetized by intraperitoneal injection of sodium pentobarbital (40 mg/kg), placed on a constant temperature hot plate at 37 ± 1°C, blood was taken by cardiac puncture or pathological sections were made to detect the side effects (gastrointestinal reaction, nervous system toxicity, hematological toxicity, allergic reaction, nephrotoxicity, hepatotoxicity, rash and asthma) The side effects of aspirin, ibuprofen, indomethacin, phenylbutazone, diclofenac, piroxicam and glucocorticoid groups were investigated according to the same administration method as compounds 1-19. The results are shown in Table 4. Compared with the traditional anti-inflammatory drugs aspirin, ibuprofen, indomethacin, phenylbutazone, diclofenac, piroxicam and glucocorticoid, the compounds 1-19 provided by the invention have the advantage pof significantly less side effects.

**Table 4 Toxicity test results**

| | Side effects | Occurrence rate of side effects |
|---|---|---|
| Aspirin | Gastrointestinal tract, nervous system, blood system, allergic reaction, nephrotoxicity, hepatotoxicity, hepatonephrotoxicity, rash and asthma | 20% |
| Ibuprofen | | |
| Indometacin | | |
| Baotaisong | | |
| Diclofenac | | |
| Piroxicam | | |

| Glucocorticoid | Intestinal tract, cardiovascular system, immune function, nervous system | 35% |
|---|---|---|
| Compounds 1,3-6,12 | Gastrointestinal tract, nervous system, blood system, allergic reaction, nephrotoxicity, hepatotoxicity, rash and asthma | 1% |
| Compounds 2,7-9 | | 1.5 % |
| Compounds 10,11,13-15 | | 3% |
| Compounds 16-19 | | 2% |

## Claims

1. A nucleoside derivative for preventing and treating inflammation, wherein the structure formula of the derivative is represented by formula (I) ~ formula (IV), or the derivative is a pharmaceutically acceptable salt of a compound of formula (I) ~ formula (IV), 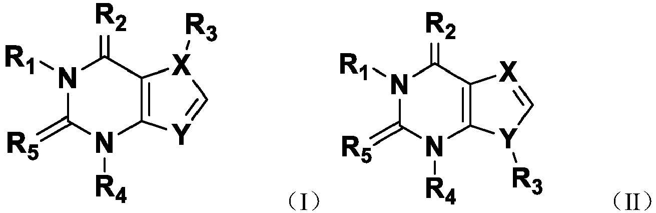 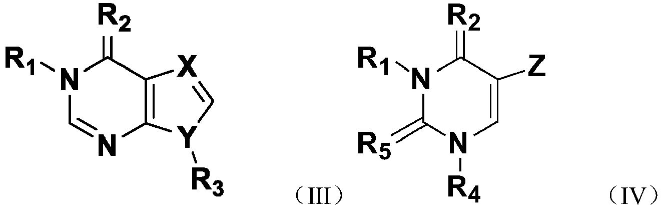 in formula (I) ~ formula (IV), R₁ is H, alkyl, heterocyclyl, alkyl substituted with an oxygen atom, a sulfur atom, a nitrogen atom or halogen, an ester group, amido or amino; R₃ and R₄ are alkyl, heterocyclyl, alkyl substituted with an oxygen atom, a sulfur atom, a nitrogen atom or halogen; R₂ and R₅ are O or S, X and Y are N or C, and Z is halogen.

2. The nucleoside derivative for preventing and treating inflammation according to claim 1, wherein in formula (I) ~ formula (IV), R₁ is H, (C₁-C₁₈) alkyl, (C₃-C₁₂) heterocyclyl, (C₁-C₁₈) alkyl substituted with an oxygen atom, a sulfur atom, a nitrogen atom or halogen, an ester group, amido or amino; R₃ and R₄ are (C₁-C₃) alkyl.

3. The nucleoside derivative for preventing and treating inflammation according to claim 2, wherein in formula (I) ~ formula (IV), R₁ is H, (C₁-C₃) alkyl, (C₃-C₁₂) heterocyclyl, (C₁-C₃) alkyl substituted with an oxygen atom, a sulfur atom, a nitrogen atom or halogen, or is a group equivalent to -A₁OC(O)A₂, -A₁NHA₂ and -A₁NHCOA₂, wherein A₁ and A₂ are (C₁-C₃) alkyl and (C₁-C₃) alkyl substituted with an oxygen atom, a sulfur atom, a nitrogen atom or halogen.

4. The nucleoside derivative for preventing and treating inflammation according to claim 3, wherein in formula (I) ~ formula (IV), R₁ is H or (C₁-C₃) alkyl, or is a group equivalent to -A₁OC(O)A₂, -A₁NHA₂ and -A₁NHCOA₂, wherein A₁ and A₂ are (C₁-C₃) alkyl.

5. The nucleoside derivative for preventing and treating inflammation according to claim 4, wherein in formula (I) ~ formula (IV), R₁ is (C₁-C₃) alkyl, or is a group equivalent to -A₁OC(O)A₂, -A₁NHA₂ and -A₁NHCOA₂, wherein A₁ and A₂ are (C₁-C₃) alkyl; in formula (I) ~ formula (IV), R₁ is H.

6. The nucleoside derivative for preventing and treating inflammation according to claim 5, wherein the nucleoside derivative is any one of the following compounds 1~19, or is a pharmaceutically acceptable salt of any one of the following compounds 1~19:

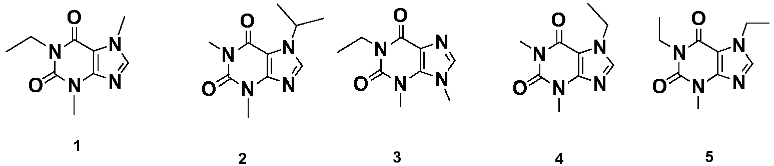

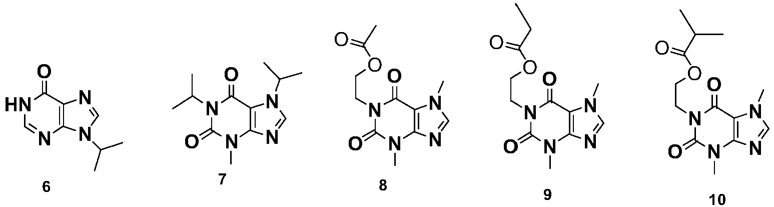

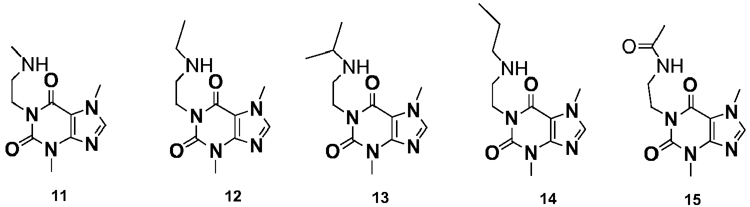

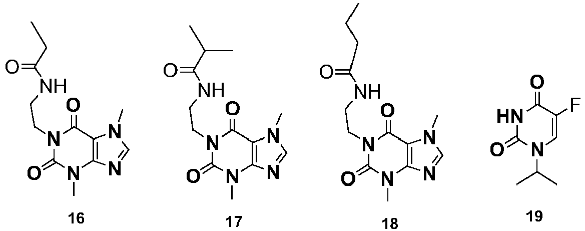

7. The nucleoside derivatives for preventing and treating inflammation according to any one of claims 1~6, wherein pharmaceutically acceptable salts of compounds of formula (I) ~ formula (IV) and a pharmaceutically acceptable salt of any one of compounds 1~19 are addition salts formed by compounds of formula (I) - formula (IV) and any one of compounds 1~19 in combination with hydrochloric acid, hydrobromic acid, sulfuric acid, carbonic acid, citric acid, succinic acid, tartaric acid, phosphoric acid, lactic acid, pyruvic acid, acetic acid, maleic acid, methanesulfonic acid, benzenesulfonic acid, p-methylbenzenesulfonic acid or ferulic acid.

8. Application of the nucleoside derivative according to any one of claims 1~7 in preparation of a drug for preventing and treating inflammatory diseases.

9. The application according to claim 8, wherein the inflammatory diseases comprise pancreatitis, hepatitis, arthritis and related complications of pancreatitis, hepatitis and arthritis.
